Europäisches Patentamt

(19) European Patent Office

Office européen des brevets

(11) Veröffentlichungsnummer: **0 065 166**
**A2**

(12) **EUROPÄISCHE PATENTANMELDUNG**

(21) Anmeldenummer: **82103751.2**

(22) Anmeldetag: **03.05.82**

(51) Int. Cl.³: **G 01 N 33/18**, G 01 N 27/28

(30) Priorität: **14.05.81 DE 3119187**

(43) Veröffentlichungstag der Anmeldung: **24.11.82**
**Patentblatt 82/47**

(84) Benannte Vertragsstaaten: **AT BE DE FR GB LU NL**

(71) Anmelder: **CILLICHEMIE Ernst Vogelmann GmbH & Co.,
Bottwarbahnstrasse 70, D-7100 Heilbronn (DE)**

(72) Erfinder: **Weller, Horst, Lerchenstrasse 19,
D-7107 Neckarsulm (DE)**

(74) Vertreter: **Patentanwälte Dr. Ing. Eugen Maier Dr. Ing.
Eckhard Wolf, Pischekstrasse 19, D-7000 Stuttgart 1 (DE)**

(54) **Vorrichtung zur Messung der chemischen Beschaffenheit von Wasser.**

(57) Bei einer Vorrichtung zur Messung der chemischen Beschaffenheit von Wasser, insbesondere von Trink-, Brauch- und Schwimmbadwasser, sind rohrförmige Durchflußmeßzellen (14, 16) für die Bestimmung des pH-Werts und/oder des Redoxpotentials sowie für die Bestimmung des Gehalts an oxydierenden Desinfektionsmitteln, wie Chlor, Brom oder Ozon, vorgesehen, die hintereinander über eine Zulaufleitung (36) mit Meßwasser beaufschlagt werden. Die beiden Meßzellen (14, 16) sind auf nach oben weisende Stecknippel (24, 28) einer Konsole (12) aufsteckbar, in der ein Teil der Verbindungsleitungen sowie ein Dreiwegeschieber (38) für die Zuschaltung eines Eichfilters (20) angeordnet sind. Der rohrförmige, mit einem Entchlorungsmittel (18) gefüllte Eichfilter (20) ist gleichfalls unmittelbar an der Konsole (12) befestigt und ist über eine innerhalb der Konsole verlaufende Leitung mit der Eintrittsseite der Chlor-Meßzelle (16) verbunden.

COMPLETE DOCUMENT

A 12 541
4.5.1981
f - kt

- 2 -

## Beschreibung

Die Erfindung betrifft eine Vorrichtung zur Messung der chemischen Beschaffenheit von Wasser, insbesondere eine Meßstation mit Meßzellen für die amperometrische Bestimmung des Gehalts an oxidierenden Desinfektionsmitteln und die potentiometrische Bestimmung des pH-Werts und des Redoxpotentials.

Die Desinfektion von Trink-, Brauch- und Schwimmbadwasser erfolgt heute überwiegend durch die Zugabe von Chlor, Chlorverbindungen, Brom oder Ozon. Hierbei kommt es vor allem auf die richtige Dosierung des Desinfektionsmittels und auf eine optimale Einstellung des pH-Werts an. Die genannten Meßzellen dienen als Meßwertgeber für die Überwachung und Regelung des Desinfektionsmittel-Gehalts und des pH-Werts. Das Redoxpotential ist eine weitere Meßgröße, die Rückschlüsse auf den Zustand und die Wirksamkeit einer Desinfektions- und Filteranlage zuläßt.

Die Meßstation mit den erforderlichen Meßzellen und Eichvorrichtungen erfordert eine Verrohrung, die bei den meisten bekannten Vorrichtungen für den Betreiber unübersichtlich ist und immer wieder zu Fehlbedienungen und Störungen vor allem bei der Wartung Anlaß gibt.

- 3 -

Der Erfindung liegt die Aufgabe zugrunde, eine Meßstation der genannten Art so zu gestalten, daß sich ein auch für den Betreiber übersichtlicher und wartungsfreundlicher Aufbau mit einem Minimum an Verrohrung ergibt.

Zur Lösung dieser Aufgabe wird die in Anspruch 1 angegebene Merkmalskombination vorgeschlagen. Weitere vorteilhafte Ausgestaltungen und Weiterbildungen der Erfindung ergeben sich aus den Unteransprüchen.

Die Erfindung geht dabei vor allem von dem Gedanken aus, daß die Verrohrung und damit die Handhabung bei der Montage und bei der Wartung dadurch übersichtlicher gestaltet werden kann, daß ein Teil der Leitungen sowie das für die Bedienung der Meßzelle zur Bestimmung des Desinfektionsmittelgehalts erforderliche Umschaltventil innerhalb einer Konsole angeordnet werden, an der außerdem geeignete Vorkehrungen für den unmittelbaren Anschluß und die Befestigung der Meßzellen und des Eichfilters vorgesehen sind. Besonders vorteilhaft in dieser Hinsicht ist die Verwendung steckbarer Anschlüsse in Form von Stecknippeln oder Steckstutzen mit entsprechenden Anschlußöffnungen im anderen Teil.

In der Zeichnung ist ein besonders vorteilhaftes Ausführungsbeispiel der erfindungsgemäßen Meßstation in schematischer Weise dargestellt. Es zeigen

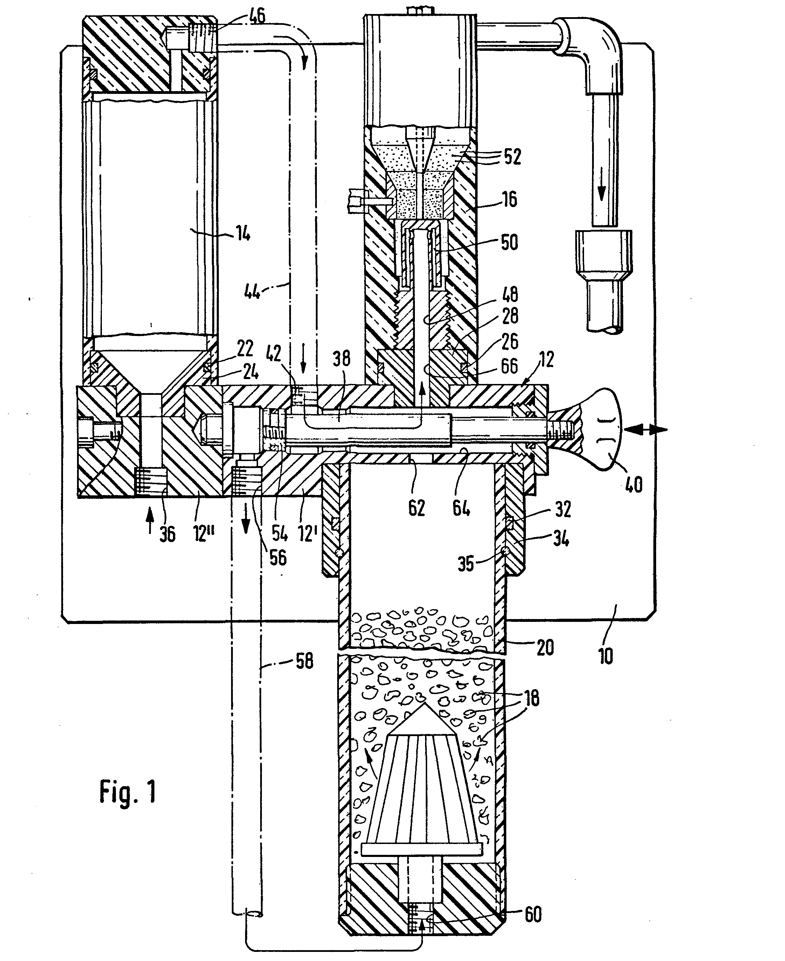

Fig. 1 eine Meßvorrichtung in senkrecht geschnittener Darstellung;

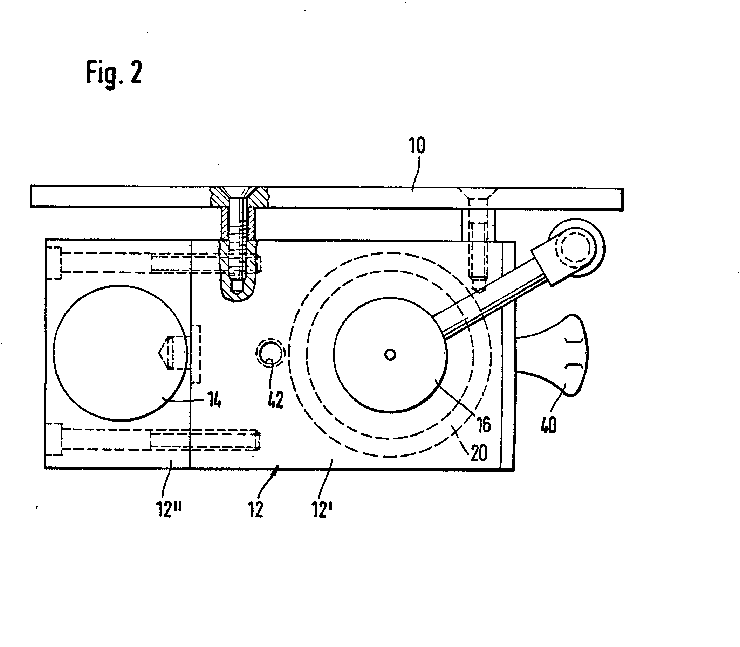

Fig. 2 eine Draufsicht auf die Meßvorrichtung nach Fig. 1.

Die in der Zeichnung dargestellte Meßstation dient zur Messung der chemischen Beschaffenheit von Wasser, insbesondere Trink-, Brauch- und Schwimmbadwasser. Sie enthält eine an einer senkrechten Montageplatte 10 befestigte Konsole 12, die eine Durchflußmeßzelle 14 mit einer Einstab-Meßkette für die potentiometrische pH-und Redoxpotentialbestimmung, eine Durchflußmeßzelle 16 für die amperometrische Bestimmung des Chlorgehalts sowie einen mit einem Entchlorungsmittel 18 gefüllten Eichfilter 20 trägt. Die Konsole 12 ist aus zwei mittels Schrauben miteinander verbundenen Teilen 12', 12" zusammengesetzt, von denen das eine Teil 12" einen nach oben weisenden, mit einem Dichtungsring 22 versehenen Stecknippel 24 zum Aufstecken der pH- und Redoxpotentialmeßzelle 14 und der andere Teil 12' einen nach oben weisenden, mit einem Dichtungsring 26 versehenen Stecknippel 28 zum Aufstecken der Chlor-Meßzelle 16 sowie einen nach unten weisenden, ebenfalls mit einem Dichtungsring 32 versehenen Steckstutzen 34 für den Eichfilter 20 enthält. Das rohrförmige Gehäuse des Eichfilters 20 ist an seinem in den Stutzen 34 eingesteckten Ende durch einen lösbaren Bügel 35 gegen ein Herausfallen oder Herausziehen formschlüssig am Stutzen verankert.

Der Rohr- oder Schlauchanschluß 36 für die Wasserzulaufleitung zur pH-Wert- und Redoxpotential-Meßzelle 14 befindet sich an der Unterseite des Konsolenteils 12''.
Innerhalb des anderen Konsolenteils 12' ist ein Umschaltventil 38 angeordnet, das als Dreiwegeschieber ausgebildet ist und über das seitlich überstehende Betätigungsorgan 40 von Hand in zwei Endstellungen verschoben werden
kann. Der die Eingangsöffnung 42 des Dreiwege-Schiebers
bildende Schlauchanschluß weist nach der Oberseite der
Konsole 12 und ist entweder über einen Schlauch 44 mit
dem Ausgang 46 der Meßzelle 14 oder, bei fehlendem Konsolenteil 12'' mit Meßzelle 14, unmittelbar mit einer
Wasserzulaufleitung verbindbar.

In der einen, in der Zeichnung gezeigten Endstellung des
Schiebers 38 gelangt das Wasser von der Eintrittsöffnung
42 unmittelbar zum Nippel 28 und von dort zur Eintrittsöffnung 48 der Chlor-Meßzelle 16. Unmittelbar hinter der
Eintrittsöffnung 48 befindet sich innerhalb der Chlor-
Meßzelle 16 ein siphonartig ausgebildeter Umlenkkanal 50,
der verhindert, daß der in der Meßzelle befindliche
Reinigungssand 52 nach unten in den Schieberbereich gelangen kann.

Zur Eichung des von der Chlor-Meßzelle 16 abgegebenen elektrischen Stroms wird das Meßwasser zunächst im Eichfilter 20 entchlort und von dort der Meßzelle 16 zugeführt. Hierzu wird der Schieber 38 in seine zweite, in der Zeichnung nach rechts verschobene Endstellung gebracht, in der der direkte Durchtritt des Meßwassers zum Nippel 46 durch den Ventilkörper 54 gesperrt und der zur Ausgangsöffnung 56 führende Strömungsweg geöffnet wird. Die Ausgangsöffnung 56 ist über einen Schlauch 58 mit der Eintrittsöffnung 60 des Eichfilters 20 verbunden, der austrittsseitig über die Bohrung 62, den Ringkanal 64 und den Nippelkanal 66 mit der darüber befindlichen Eintrittsöffnung 48 der Chlor-Meßzelle hydraulisch verbunden ist.

DR.-ING. EUGEN MAIER    DR.-ING. ECKHARD WOLF

PATENTANWALTE

ZUGELASSENE VERTRETER VOR DEM EUROPÄISCHEN PATENTAMT

TELEFON: (0711) 24 27 61/2
TELEGRAMME: MENTOR

7 STUTTGART 1, PISCHEKSTR. 19

DRESDNER BANK AG
STUTTGART NR. 1 920 534
POSTSCHECK STGT. 25200-709

- 7 -

A 12 541
4. Mai 1981
f - kt

CILLICHEMIE
Ernst Vogelmann GmbH &  Co.

Bottwarbahnstr. 70

7100 Heilbronn

---

Vorrichtung zur Messung der chemischen
Beschaffenheit von Wasser

---

A n s p r ü c h e

1. Vorrichtung zur Messung der chemischen Beschaffenheit
von Wasser mit einer eintrittsseitig mittels eines Umschaltventils (38) wahlweise direkt oder über einen Eichfilter (20)
mit einer Zulaufleitung verbindbaren Durchflußmeßzelle (16)
für die Bestimmung des Gehalts an oxidierenden Desinfektionsmitteln, wie Chlor, Brom oder Ozon, d a d u r c h  g e -
k e n n z e i c h n e t , daß das Umschaltventil (38)
innerhalb einer an einer senkrechten Wand oder Montageplatte (10) befestigbaren, die Meßzelle (16) und den Eichfilter (20) tragenden Konsole (12) angeordnet ist, die
einen nach oben weisenden, den einen Ausgang des Umschaltventils (38) bildenden Nippel (46) für die unmittelbare Befestigung der eintrittsseitig mit einer entsprechenden, nach unten weisenden Anschlußöffnung versehenen Meßzelle, einen nach unten weisenden, mit dem Nippel (46) innerhalb der Konsole (12) hydraulisch verbundenen Stutzen (34) zum unmittelbaren Befestigen des Eichfilters (20) mit seinem nach oben weisenden austrittsseitigen Anschluß sowie einen den Eingang (42) des Umschaltventils (38) bildenden Rohr- oder Schlauchanschluß für die Zulaufleitung und einen den zweiten Ausgang (56) des Umschaltventils bildenden Rohr- oder Schlauchanschluß für eine zum eintrittsseitigen Anschluß (60) des Eichfilters (20) führende Leitung (58) enthält.

2. Vorrichtung nach Anspruch 1, mit einer in der Zulaufleitung vor dem Umschaltventil (38) angeordneten zweiten Durchflußmeßzelle (14) für die Bestimmung des pH-Werts und/oder des Redox-Potentials, d a d u r c h   g e k e n n - z e i c h n e t , daß die Konsole (12) an einem gegebenenfalls lösbar befestigten Teil (12") einen zweiten, nach oben weisenden Nippel (24) zum unmittelbaren Befestigen der eintrittsseitig mit einer entsprechenden, nach unten weisenden Anschlußöffnung versehenen zweiten Meßzelle sowie einen innerhalb des betreffenden Konsolteils (12") zu diesem Nippel(24) führenden Rohr- oder Schlauchanschluß (36) für die Zulaufleitung enthält, und daß der die Eintrittsöffnung (42) des Umschaltventils (38) bildende Rohr- oder Schlauchanschluß über eine Flüssigkeitsleitung mit dem Ausgang der zweiten Meßzelle (14)
verbunden ist.

3. Vorrichtung nach Anspruch 1 oder 2, d a d u r c h
g e k e n n z e i c h n e t , daß zumindest einer der
Nippel (24,28) als mit einem Dichtungsring (22) versehener
Stecknippel ausgebildet ist, auf den die betreffende Meßzelle (14,16) mit ihrer buchsenartig ausgebildeten Anschlußöffnung aufsteckbar ist.

4. Vorrichtung nach einem der Ansprüche 1 bis 3, d a d u r c h
g e k e n n z e i c h n e t , daß der austrittsseitige
Anschluß des Eichfilters (20) als auf oder in den an der
Konsole (12) nach unten weisenden Stutzen (34) auf- oder
einsteckbares und dort gegen ein Herausziehen und Herausfallen arretierbares Rohrstück ausgebildet ist.

5. Vorrichtung nach einem der Ansprüche 1 bis 4, d a -
d u r c h g e k e n n z e i c h n e t , daß mindestens
eine der Meßzellen (14) eintrittsseitig einen syphonartig
ausgebildeten Umlenkkanal (50) enthält.

6. Vorrichtung nach einem der Ansprüche 1 bis 5, d a -
d u r c h g e k e n n z e i c h n e t , daß das Umschaltventil (38) als an einem seitlich über die Konsole überstehenden Betätigungsorgan (40) von Hand verstellbarer
Dreiwegeschieber ausgebildet ist.

Fig. 1

Cillichemie Ernst Vogelmann GmbH u.Co
Patentanwälte Dr.-Ing. Eugen Maier – Dr.-Ing Eckhard Wolf
Pischekstraße 19 – 7000 Stuttgart 1·

A 12 541

Fig. 2

Cillichemie Ernst Vogelmann GmbH u.Co
Patentanwälte Dr.-Ing. Eugen Maier – Dr.-Ing. Eckhard Wolf          A 12 541
Pischekstraße 19 - 7000 Stuttgart 1